# EUROPEAN PATENT APPLICATION

(11) **EP 4 684 765 A1**
(43) Date of publication of application: **28.01.2026**
(21) Application number: 24382817.5
(22) Date of filing: 25.07.2024
(51) Int. Cl.: A61F 2/90, B29C 64/106, B29C 64/241, B29C 64/245, B29L 31/00, B33Y 10/00, B33Y 80/00, B33Y 30/00, B33Y 70/00

(54) **METHOD OF MANUFACTURING A BIODEGRADABLE POLYMERIC INTRAVASCULAR TUBULAR DEVICE USING A 3D PRINTER AND BIODEGRADABLE POLYMERIC INTRAVASCULAR TUBULAR DEVICE**

(71) Applicant: Universitat Politècnica De Catalunya, 08034 Barcelona (ES)
(72) Inventor: PEGUEROLES NEYRA, Marta, 08012 Barcelona (ES); CHAUSSE CALBET, Víctor, 43850 Cambrils (ES); CANO MORENILLA, Mariola, 30800 Lorca (ES)
(74) Representative: Torner, Juncosa I Associats, SL

(57) **Abstract**

A method of manufacturing a biodegradable polymeric intravascular tubular device using a 3D printer and a biodegradable polymeric intravascular tubular device are provided. The method comprises patterning a linear micropattern design comprising different periodicities ranging from 3 µm to 32 µm and a given depth onto a surface of a steel rod; dispensing, while the steel rod is rotating, a polymer-based ink on the generated linear micropattern using a nozzle of a 3D printer; and obtaining a biodegradable intravascular device with an inner biomimetic topography through solidification of the polymer-based ink.

## Description

### TECHNICAL FIELD

The present invention relates to medical devices and manufacturing methods thereof. Particularly, the present invention relates to a method of manufacturing a biodegradable polymeric intravascular tubular device using a 3D printer and to a biodegradable polymeric intravascular tubular device.

### BACKGROUND OF THE INVENTION

Biodegradable intravascular tubular devices, such as bioresorbable stents (BRS), show potential for treating coronary heart disease, particularly for patients with special needs such as small vessel diameter, children's diseases or diabetes. While traditional stent fabrication technologies, such as stent braiding or laser machining, continue to advance, three-dimensional (3D) printing presents a promising alternative for personalized medical solutions. Although producing unique parts can be costly and time-consuming, the integration of 3D printing with existing 3D imaging techniques in the healthcare industry enhances its viability for patient benefits

Among 3D Printing techniques, nozzle-based deposition involves continuously applying ink through a nozzle to create a 3D pattern layer-by-layer. The ink may be obtained by polymer melting, known as fused-deposition modeling (FDM), or by dissolving the polymer in a solvent and extruding it through pressure assisted microsyringes, known as solvent-cast direct-writing (SC-DW).

There are known some patents and patent applications providing stents with some sort of topographic coating. For instance, CN115583017 (A) provides a micro-nano 3D printing surface coated conductive biological scaffold and a preparation method thereof. The preparation method comprises the following steps: designing a biological scaffold structure model; selecting an insulating hard material as a substrate, and pretreating the substrate to reduce the surface energy of the substrate for later use; selecting a biodegradable high-molecular polymer as a printing material, and printing a biological scaffold with a preset pattern on a substrate by adopting an electric field driven spray deposition micro-nano 3D printing technology; and a conductive material solution with the polarity opposite to that of the printing voltage is selected to coat the surface of the stent, and the surface of the biological stent is uniformly wrapped with the conductive material through the electrostatic attraction effect of the charged biological stent and the conductive material, so that the conductive biological stent with high conductivity is obtained.

Another example is WO2005099621-A1, which provides implantable medical devices coated with topographic coatings and methods for the manufacture and use thereof. The topographic coatings are jet printed onto the surface of the medical device, creating regions of high elevation and low elevation in a predetermined pattern.

However, none of these prior art solutions provides for a medical device with an inner controlled biomimetic topography obtained through surface physical modification of a steel rod and subsequent 3D printing of the device, that could accelerate endothelium healing by increasing endothelial cells (ECs) adhesion and migration onto stent surfaces. Consequently, there is a need for new manufacturing methods and intravascular medical devices that address these issues.

### DESCRIPTION OF THE INVENTION

To that end, an object of present invention is thus to provide a manufacturing method for a biodegradable polymeric intravascular tubular device using a 3D printer, and a biodegradable polymeric intravascular tubular device, specifically, for treating cardiovascular diseases in children and adults that enhances EC migrations and improves device tissue integration within the vessel.

This object is fulfilled by a method with the characteristics of claim 1 and by a device with the features of claim 10.

According to one aspect, a method of manufacturing a biodegradable polymeric intravascular tubular device (e.g. a stent) using a 3D printer is proposed. The method comprises patterning a linear micropattern design comprising different periodicities ranging from 3 µm to 32 µm and a given depth onto a surface of a steel rod; dispensing, while the steel rod is rotating, a polymer-based ink on the generated linear micropattern using a nozzle of a 3D printer; and obtaining a biodegradable intravascular device with an inner biomimetic topography through solidification of the polymer-based ink.

According to another aspect, a biodegradable polymeric intravascular tubular device is proposed. The device comprises a tubular member made of a polymer-based ink and including an inner biomimetic topography having a linear micropattern design with different periodicities ranging from 3 µm to 32 µm and a given depth.

That is, the device is manufactured/printed with optimized inner linear patterning periodicities and depths, specifically designed according to EC dimensions, to accelerate tissue regeneration.

In some embodiments, the patterning is carried out by a laser applying a direct laser interference patterning (DLIP) technique. Alternatively, in other embodiments, it is performed through an extrusion method or electrospinning.

In some embodiments, the given depth ranges between 500 and 1000 nm.

In some embodiments, the polymer-based ink is obtained by dissolving different thermoplastic polymers with a solvent following a solvent-cast direct-writing (SC-DW) procedure. The thermoplastic polymers can comprise poly-I-lactic acid (PLLA), poly(I-lactic-co-*ε-*caprolactone) (PLCL) and/or PLLA/PLCL blends.

In some embodiments, the polymer-based ink has shape-memory properties.

In some embodiments, the polymer-based ink further comprises one or more radiopaque agents, one or more drug agents and/or one or more functionalization agents.

In some embodiments, an outlet aperture of the nozzle is comprised between 180 µm and 275 µm.

Present invention allows for the creation of polymeric tubular constructs through solvent-cast direct-ink writing using a modified 3D printer equipped with a rotating steel rod, or mandrel. This setup enables the production of various designs and diameter sizes. Additionally, radiopaque agents or drugs can be incorporated into the polymeric ink before printing, and printing onto the patterned steel rod allows for the creation of inner topographies that enhance tissue regeneration. The applications of this technology span across various medical fields where tubular constructs are required to open vessels. Examples include cardiovascular stents for arteries, aortic stents, treatment for peripheral artery disease, urinary stents, or as support for neurovascular grafts. Furthermore, the device can be crimped onto a balloon for insertion within a catheter during minimally invasive surgery.

### BRIEF DESCRIPTION OF THE DRAWINGS

The previous and other advantages and features will be more fully understood from the following detailed description of embodiments, with reference to the attached figures, which must be considered in an illustrative and non-limiting manner, in which:
Fig. 1 illustrates the design scheme of a biodegradable polymeric intravascular tubular device and printing setup. The design in the XY plane is printed as a mono layer onto the rotating steel rod surface.
Fig. 2 shows an optimized linear patterning for ECs migration and device tissue integration with the vessel.
Fig. 3 illustrates different examples of biodegradable polymeric intravascular tubular devices obtained by the proposed method.

### DETAILED DESCRIPTION OF THE INVENTION AND OF PREFERRED EMBODIMENTS

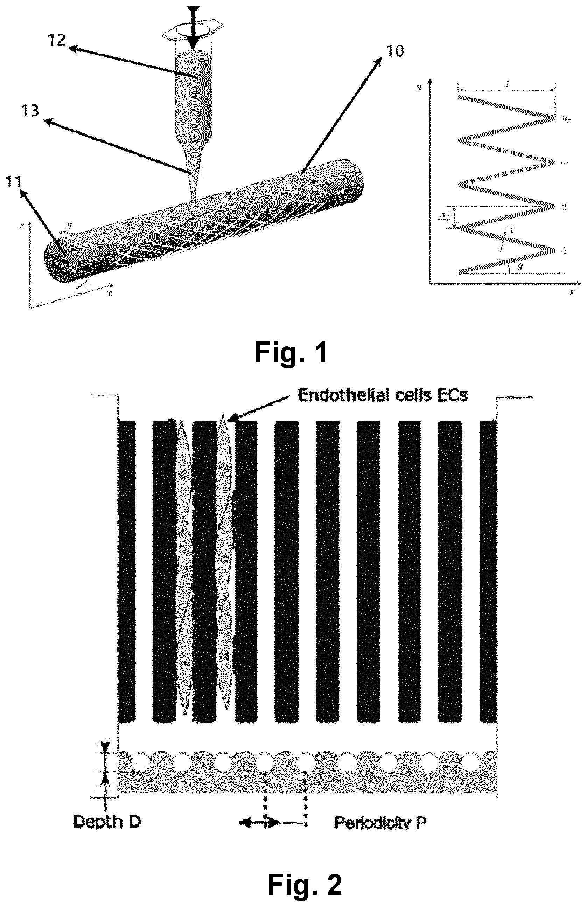

With reference to Fig. 1, an embodiment of the proposed manufacturing method is schematically illustrated. In this embodiment, an intravascular tubular device 20, particularly a stent, is fabricated using the solvent-cast direct-writing (SC-DW) technique, wherein a polymer-based ink 12 is extruded through a nozzle 13.

To provide the biomimetic topography to the inner surface of the intravascular tubular device 20, a steel rod 11 is previously patterned with a linear micropattern design 10. This linear design can be created using computer-assisted technologies, such a CAD file or the like. The inner biomimetic topography is manufactured/printed to have optimized inner linear patterning periodicities and depths, to enhance EC migration and improve stent tissue integration with the vessel.

In some embodiments, the steel rod 11 is patterned using direct laser interference patterning (DLIP). Patterns with different periodicities ranging from 3 µm to 32 µm can be generated by adjusting the angle (Fig 2.). In some cases, the patterns comprise four different periodicities. Additionally, to achieve the desired depths, laser fluency can be varied to induce differences in the structure depth. For instance, fluencies inferior to 1 J cm⁻² can be applied to generate low-depth structures of <40 nm (L-pattern) and fluencies superior to 1.65 J cm⁻² can be used to obtain high-depth structures of >600 nm (H-pattern).

In other embodiments, other conventional fabrication methods, such as extrusion, electrospinning, or die modification, can also be used for patterning.

Continuing with the explanation of Fig. 1, once the steel rod 11 is patterned, the polymer-based ink 12 is dispensed over it while it rotates. This process results in the formation of the intravascular tubular device 20 with an inner biomimetic topography, featuring diameters ranging from 2 mm to 10 mm, achieved through the solidification of the polymer-based ink 12.

The polymer-based ink 12 can comprise any of the following: poly-I-lactic acid (PLLA), poly(l-lactic-co-*ε*-caprolactone) (PLCL) and/or PLLA/PLCL blends. In some embodiments, the ink is prepared by dissolving of PLLA or PLCL pellets in chloroform at ratios from 5% to 25% (w/v). Polymer dissolution can be ensured by means of a dual asymmetric centrifuge, and the obtained ink can be introduced into cartridges. Different nozzles 13 can be used for extrusion, typically using from 180 µm to 275 µm, allowing for device strut thicknesses to range from 80 µm up to 200 µm, depending on the polymer ink composition and nozzle diameters.

In some embodiments, the device 20 can exhibit shape-memory properties, depending on the polymer ink composition used. Alternatively or complementarily, the polymer-based ink 12 can be tuned with additional features, such as radiopaque agents, drugs or functionalization, by adding these agents to the polymeric ink solution in chloroform.

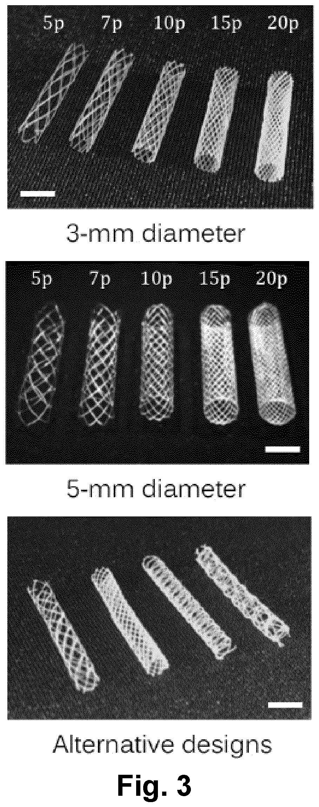

Fig. 3 shows some examples of biodegradable polymeric intravascular tubular devices 20 that can be obtained using the proposed method.

Terminology used herein is for the purpose of describing particular embodiments only and is not intended to be limiting of the invention. For example, as used herein, the singular forms "a", "an" and "the" are intended to include the plural forms as well, unless the context clearly indicates otherwise. It will be further understood that the terms "comprises" and/or "comprising," when used in this specification, specify the presence of stated features, steps, operations, elements, and/or components, but do not preclude the presence or addition of one or more other features, steps, operations, elements, components, and/or groups thereof. As used herein, the term "and/or" includes any and all combinations of one or more of the associated listed items and may be abbreviated as "/".

The embodiments and illustrations included herein show, by way of illustration and not of limitation, specific embodiments in which the subject matter may be practiced. Other embodiments may be utilized and derived there from, such that structural and logical substitutions and changes may be made without departing from the scope of this disclosure. Such embodiments of the inventive subject matter may be referred to herein individually or collectively by the term "invention" merely for convenience and without intending to voluntarily limit the scope of this application to any single invention or inventive concept, if more than one is, in fact, disclosed. Combinations of the above embodiments, and other embodiments not specifically described herein, will be apparent to those of skill in the art upon reviewing the above description.

The scope of the present invention is defined in the following set of claims.

## Claims

1. A method of manufacturing a biodegradable polymeric intravascular tubular device using a 3D printer, comprising:
patterning a linear micropattern design (10) comprising different periodicities ranging from 3 µm to 32 µm and a given depth onto a surface of a steel rod (11);
dispensing, while the steel rod (11) is rotating, a polymer-based ink (12) on the generated linear micropattern (10) using a nozzle (13) of a 3D printer; and
obtaining a biodegradable polymeric intravascular tubular device (20) with an inner biomimetic topography through solidification of the polymer-based ink (12).

2. The method of claim 1, wherein the patterning is performed by a laser applying a direct laser interference patterning, DLIP, technique.

3. The method of claim 1, wherein the patterning is performed by means of an extrusion method or by electrospinning.

4. The method of any one of the previous claims, wherein the given depth ranges between 500 and 1000 nm.

5. The method of any one of the previous claims, wherein the polymer-based ink (12) is obtained by dissolving different thermoplastic polymers with a solvent following a solvent-cast direct-writing, SC-DW, procedure, the thermoplastic polymers comprising poly-I-lactic acid, PLLA, poly(I-lactic-co-*ε*-caprolactone), PLCL, and/or PLLA/PLCL blends.

6. The method of any one of the previous claims, wherein the polymer-based ink (12) has shape-memory properties.

7. The method of any one of the previous claims, wherein the polymer-based ink (12) further comprises at least one of: a radiopaque agent, a drug agent, and a functionalization agent.

8. The method of any one of the previous claims, wherein an outlet aperture of the nozzle (13) is comprised between 180 µm and 275 µm.

9. The method of any one of the previous claims, wherein the biodegradable polymeric intravascular tubular device (20) comprises a stent.

10. A biodegradable polymeric intravascular tubular device, comprising a tubular member made of a polymer-based ink and comprising an inner biomimetic topography having a linear micropattern design with different periodicities ranging from 3 µm to 32 µm and a given depth.

11. The device of claim 10, wherein the given depth ranges between 500 and 900 nm.

12. The device of claim 10 or 11, wherein the polymer-based ink is made of thermoplastic polymers including poly-I-lactic acid, PLLA, poly(I-lactic-co-*ε*-caprolactone), PLCL, and/or PLLA/PLCL blends.

13. The device of any one of the previous claims 10-12, wherein the polymer-based ink has shape-memory properties.

14. The device of any one of the previous claims 10-13, wherein the polymer-based ink further comprises at least one of: a radiopaque agent, a drug agent, and a functionalization agent.

15. The device of any one of the previous claims 10-14, comprising a stent.
